(19)

**Europäisches Patentamt**
**European Patent Office**
**Office européen des brevets**

(11) **EP 4 179 467 B1**

(12) **EUROPEAN PATENT SPECIFICATION**

(45) Date of publication and mention
of the grant of the patent:
**17.12.2025 Bulletin 2025/51**

(21) Application number: **21742110.6**

(22) Date of filing: **08.07.2021**

(51) International Patent Classification (IPC):
*G16H 30/40* (2018.01)     *G16H 50/20* (2018.01)
*G06N 3/084* (2023.01)     *G06N 20/10* (2019.01)
*G06N 20/20* (2019.01)     *G06N 3/045* (2023.01)

(52) Cooperative Patent Classification (CPC):
**G16H 30/40; G06N 3/0464; G06N 3/084;
G06N 3/09; G06N 3/091; G06N 3/098; G06N 20/10;
G06N 20/20; G16H 50/20;** G06N 3/045

(86) International application number:
**PCT/EP2021/068922**

(87) International publication number:
**WO 2022/008630 (13.01.2022 Gazette 2022/02)**

(54) **TRAINING A MODEL TO PERFORM A TASK ON MEDICAL DATA**

TRAINIEREN EINES MODELLS ZUR DURCHFÜHRUNG EINER AUFGABE AUF MEDIZINISCHEN
DATEN

FORMATION D'UN MODÈLE POUR EFFECTUER UNE TÂCHE SUR DES DONNÉES MÉDICALES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.07.2020 EP 20185311**

(43) Date of publication of application:
**17.05.2023 Bulletin 2023/20**

(73) Proprietor: **Koninklijke Philips N.V.
5656 AG Eindhoven (NL)**

(72) Inventors:
• **PATIL, Ravindra, Balasaheb**
**5656 AE Eindhoven (NL)**
• **KULKARNI, Chaitanya**
**5656 AE Eindhoven (NL)**
• **MYSORE SIDDU, Dinesh**
**5656 AE Eindhoven (NL)**
• **PANDYA, Maulik, Yogeshbhai**
**5656 AE Eindhoven (NL)**

(74) Representative: **Philips Intellectual Property &
Standards
High Tech Campus 52
5656 AG Eindhoven (NL)**

(56) References cited:
**EP-A1- 3 528 179**

• **SEYYEDALI HOSSEINALIPOUR ET AL: "From
Federated Learning to Fog Learning: Towards
Large-Scale Distributed Machine Learning in
Heterogeneous Wireless Networks",
ARXIV.ORG, CORNELL UNIVERSITY LIBRARY,
201 OLIN LIBRARY CORNELL UNIVERSITY
ITHACA, NY 14853, 7 June 2020 (2020-06-07),
XP081692346**
• **WIKIPEDIA: "Federated learning", 28 June 2020
(2020-06-28), XP055749944, Retrieved from the
Internet <URL:https://en.wikipedia.org/w/index.
php?title=Federated_learning&
oldid=964899708> [retrieved on 20201112]**

**EP 4 179 467 B1**

## Description

### FIELD OF THE INVENTION

Embodiments herein relate to training a model using a distributed machine learning process.

### BACKGROUND OF THE INVENTION

Learning from large volumes of patient data can greatly increase capacity to generate and test hypotheses about healthcare. To capture and use the knowledge contained in large volumes of patient data, predictive models are used. Models can be trained using machine learning processes on large volumes of data from patients who have been treated previously. Models trained in this manner have the potential to be used to make predictions in many areas of medicine, such as image segmentation and diagnosis, amongst others. Such models may be used to better personalise healthcare.

One of the major hurdles in enabling personalised medicine through the use of models trained using machine learning processes, is obtaining sufficient patient data to train the models. The data from one single hospital is unlikely to be sufficient to develop models which can be used on a wide variety of patients (e.g. which may be spread across the globe). However, to get the data from different hospitals and patient groups can take a long time which increases the time from planning to deployment of the models. In the Deep Learning domain, the performance of models improves with increasing numbers of training data samples. Thus, to ensure the best possible models to aid physicians, the performance of the models can be actively improved with more data. Combining data originating from multiple clinical sites (e.g. hospitals, doctors' surgeries etc) can be difficult however due to ethical, legal, political, and administrative barriers associated with data sharing. One way of mitigating such issues is by training a model using a distributed machine learning process, such as, for example, a Federated Learning process such as that described in the paper by Bonawitz et al. 2019 entitled "Towards Federated Learning at Scale: System Design". Distributed learning enables models to be trained using data from different clinical sites without the data leaving the premises.

Document EP 3 528 179 A1 relates to training a neural network for classifying medical data. A central processor acts to collate weight values and thus learning insights from a plurality of local instances of a neural network, without the medical data used to train the local neural networks needing to be transferred or combined into a single database. The local instances of the neural network are combined by using a weighted average.

### SUMMARY OF THE INVENTION

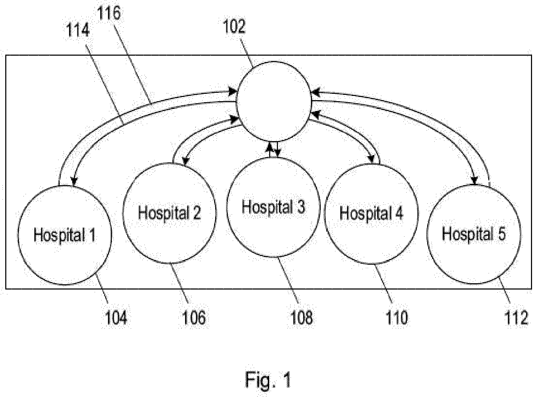

As noted above, distributed machine learning processes can be used to train models (otherwise known as "machine learning models") on training data located at different sites, without the training data needing to be moved from the respective sites. The skilled person will be familiar with distributed learning and distributed learning processes such as federated machine learning, however, this is illustrated briefly in Fig. 1 which shows a central server 102 in communication with a plurality of clinical sites, 104 to 112. The central server co-ordinates training of a model using a distributed learning process using training data located at each of the clinical sites 104 to 112. The central server holds a "global" or central copy of the model and may send 114 information about the global model, e.g. such as parameters enabling a local copy of the model to be created, to each clinical site. Each clinical site may then create a local copy of the model and train its local copy on training data at the respective clinical site. Each clinical site 104 to 112 may then send 116 an update to one or more parameters of the model to the central server. The central server combines the updates, for example through averaging, from the respective clinical sites to update the global model. This allows a global model at a central server 102 to be trained e.g. updated and improved, based on training data at a plurality of clinical sites 104 to 112, without the data having to leave the respective clinical site. It is an object of embodiments herein to improve on such processes for training models to perform a task on medical data using distributed machine learning process.

Thus according to a first aspect there is a method of training a model to perform a task on medical data using a distributed machine learning process whereby a global model is updated based on training performed on local copies of the model at a plurality of clinical sites. The method comprises: a) sending information to the plurality of clinical sites to enable each of the plurality of clinical sites to create a local copy of the model and train the respective local copy of the model on training data at the respective clinical site; b) receiving, from each of the plurality of clinical sites, i) a local update to a parameter in the model obtained by training the local copy of the model on the training data at the respective clinical site and ii) metadata related to a quality of the training performed at the respective clinical site; and c) updating the parameter in the global model, based on the received local updates to the parameter and the received metadata.

Thus, metadata related to the quality of the training performed at each site may be used when combining the local updates into an update for the global model. In this way, different local updates may be given different significances (e.g. through the use of weightings) dependent on the quality of the training performed at the respective clinical site. This can result in improved training, resulting in improved models and thus improved clinical outcomes for clinical processes that use the models. Since the model is trained on data from different sites, there may be irregularities in the data, and this can lead to bias and model drift. By considering appropriate metadata while merging the weights, model drift may be avoided, leading to a better quality model.

According to a second aspect there is a method at a clinical site for training a model to perform a task on medical data using a distributed machine learning process whereby a global model at a central server is updated based on training performed on a local copy of the model at the clinical site. The method comprises: receiving information from a central server enabling a local copy of the model to be created and trained on training data at the clinical site; training a local copy of the model according to the information; and sending to the central server, i) an update to the model based on training of the local copy of the model on the training data at the clinical site and ii) metadata related to a quality of the training performed at the respective clinical site.

According to a third aspect there is the use of a model trained according to the first or second aspects to perform a task on medical data.

According to a fourth aspect there is an apparatus for training a model to perform a task on medical data using a distributed machine learning process whereby a global model is updated based on training performed at a plurality of clinical sites. The apparatus comprises a memory comprising instruction data representing a set of instructions, and a processor configured to communicate with the memory and to execute the set of instructions. The set of instructions, when executed by the processor, cause the processor to: a) send information to the plurality of clinical sites to enable each of the plurality of clinical sites to create a local copy of the model and train the respective local copy of the model on training data at the respective clinical site; b) receive, from each of the plurality of clinical sites, i) a local update to a parameter in the model obtained by training the local copy of the model on the training data at the respective clinical site and ii) metadata related to a quality of the training performed at the respective clinical site; and c) update the parameter in the global model, based on the received local updates to the parameter and the received metadata.

According to a fifth aspect there is a computer program product comprising a computer readable medium, the computer readable medium having computer readable code embodied therein, the computer readable code being configured such that, on execution by a suitable computer or processor, the computer or processor is caused to perform the method of the first and second aspects.

These and other aspects will be apparent from and elucidated with reference to the embodiment(s) described hereinafter.

BRIEF DESCRIPTION OF THE DRAWINGS

Example embodiments will now be described, by way of example only, with reference to the following drawings, in which:

Fig. 1 illustrates a distributed learning process for training a model;
Fig. 2 illustrates an apparatus according to some embodiments herein;
Fig. 3 illustrates a method according to some embodiments herein;
Fig. 4 illustrates a method of determining model drift according to some embodiments herein;
Fig. 5 illustrates an apparatus according to some embodiments herein;

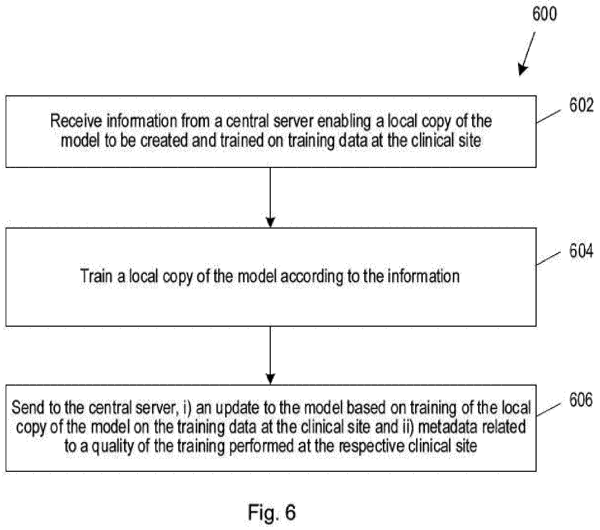

Fig. 6 illustrates a method according to some embodiments herein;
Fig. 7 illustrates a system according to some embodiments herein; and
Fig. 8 shows a segmentation of an image of a liver according to a model trained according an embodiment herein.

DETAILED DESCRIPTION OF EMBODIMENTS

As described above, embodiments herein aim to improve methods for training clinical models to perform a task on medical data using distributed machine learning processes.

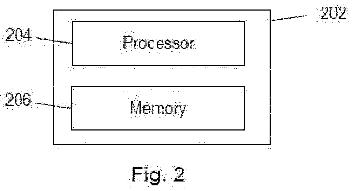

Turning to Fig. 2, in some embodiments there is an apparatus 200 for use in training a model to perform a task on medical data using a distributed machine learning process, according to some embodiments herein. Generally, the apparatus may form part of a computer apparatus or system e.g. such as a laptop, desktop computer or other computing device. In some embodiments, the apparatus 200 may form part of a distributed computing arrangement or the cloud.

The apparatus comprises a memory 204 comprising instruction data representing a set of instructions and a processor 202 (e.g. processing circuitry or logic) configured to communicate with the memory and to execute the set of instructions. Generally, the set of instructions, when executed by the processor, may cause the processor to perform any of the embodiments of the method 300 as described below.

Embodiments of the apparatus 200 may be for use in training a model to perform a task on medical data using a distributed machine learning process whereby a global model is updated based on training performed on local copies of the model at a plurality of clinical sites. More specifically, the set of instructions, when executed by the processor 202, cause the processor to: a) send information to the plurality of clinical sites to enable each of the plurality of clinical sites to create a local copy of the model and train the respective local copy of the model on training data at the respective clinical site; b) receive, from each of the plurality of clinical sites, i) a local update to a parameter in the model obtained by training the local copy of the model on the training data at the respective clinical site and ii) metadata related to a quality of the training performed at the respective clinical site; and c) update the parameter in the global model, based on the received local updates to the parameter and the received metadata.

The processor 202 can comprise one or more processors, processing units, multi-core processors or modules that are configured or programmed to control the apparatus 200 in the manner described herein. In particular implementations, the processor 202 can comprise a plurality of software and/or hardware modules that are each configured to perform, or are for performing, individual or multiple steps of the method described herein. The processor 202 can comprise one or more processors, processing units, multi-core processors and/or modules that are configured or programmed to control the apparatus 200 in the manner described herein. In some implementations, for example, the processor 202 may comprise a plurality of (for example, interoperated) processors, processing units, multi-core processors and/or modules configured for distributed processing. It will be appreciated by a person skilled in the art that such processors, processing units, multi-core processors and/or modules may be located in different locations and may perform different steps and/or different parts of a single step of the method described herein.

The memory 204 is configured to store program code that can be executed by the processor 202 to perform the method described herein. Alternatively or in addition, one or more memories 204 may be external to (e.g. separate to or remote from) the apparatus 200. For example, one or more memories 204 may be part of another device. Memory 204 can be used to store the global model, the received local updates, the received metadata and/or any other information or data received, calculated or determined by the processor 202 of the apparatus 200 or from any interfaces, memories or devices that are external to the apparatus 200. The processor 202 may be configured to control the memory 204 to store the global model, the received local updates, the received metadata and/or the any other information or data described herein.

In some embodiments, the memory 204 may comprise a plurality of sub-memories, each sub-memory being capable of storing a piece of instruction data. For example, at least one sub-memory may store instruction data representing at least one instruction of the set of instructions, while at least one other sub-memory may store instruction data representing at least one other instruction of the set of instructions.

It will be appreciated that Fig. 2 only shows the components required to illustrate this aspect of the disclosure and, in a practical implementation, the apparatus 200 may comprise additional components to those shown. For example, the apparatus 200 may further comprise a display. A display may comprise, for example, a computer screen, and/or a screen on a mobile phone or tablet. The apparatus may further comprise a user input device, such as a keyboard, mouse or other input device that enables a user to interact with the apparatus, for example, to provide initial input parameters to be used in the method described herein. The apparatus 200 may comprise a battery or other power supply for powering the apparatus 200 or means for connecting the apparatus 200 to a mains power supply.

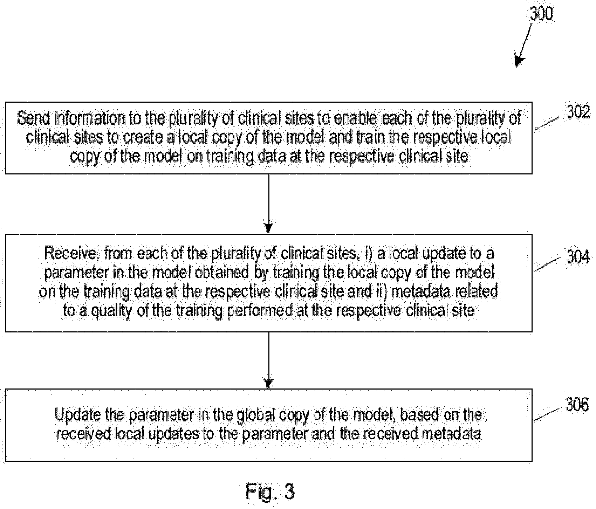

Turning to Fig. 3, there is a computer implemented method 300 for use in training a model to perform a task on (e.g. process) medical data using a distributed machine learning process whereby a global model is updated based on training performed on local copies of the model at a plurality of clinical sites. Embodiments of the method 300 may be performed, for example by an apparatus such as the apparatus 200 described above.

Briefly, in step a), the method 300 comprises: sending 302 information to the plurality of clinical sites to enable each of the plurality of clinical sites to create a local copy of the model and train the respective local copy of the model on training data at the respective clinical site. In step b) the method 300 comprises receiving 304, from each of the plurality of clinical sites, i) a local update to a parameter in the model obtained by training the local copy of the model on the training data at the respective clinical site and ii) metadata related to a quality of the training performed at the respective clinical site. In step c) the method comprises updating 306 the parameter in the global model, based on the received local updates to the parameter and the received metadata.

As noted above, since the model is trained on data from different sites, there may be irregularities in the data between sites, and this can lead to bias and model drift, whereby there is a divergence of a decision boundary used to perform the task (e.g. classify/segment etc) between different epochs of training. Generally, bias describes how well a model matches the training set. A model with high bias won't match the data set closely, while a model with low bias will match the data set very closely. Bias comes from models that are overly simple and fail to capture the trends present in the data set. Model drift can be classified into two broad categories. The first type is called 'concept drift'. Concept drift means that the statistical properties of the target variable, which the model is trying to predict, change over time in unforeseen ways. This causes problems because the predictions become less accurate as time passes. 'Data drift' : if the underlying variables are changing, the model is bound to fail. This happens when the statistical properties of the predictors change.

By considering appropriate metadata while merging the weights, model drift may be avoided, leading to a better quality model. Thus, metadata may be used related to the quality of the training performed at each site when combining the local updates into an update for the global model. In this way, different local updates may be given different significances (e.g. through the use of weightings) dependent on the quality of the training performed at the respective clinical site.

In more detail, the model may comprise any type of model that can be trained using a machine learning process. Examples of models include, but are not limited to neural networks, deep neural networks such as F-Nets, U-Nets and Convolutional Neural Networks, Random Forest models and Support Vector Machine (SVM) models.

The skilled person will be familiar with machine learning and machine learning models, but briefly, machine learning can be used to find a predictive function for a given dataset; the dataset is typically a mapping between a given input to an output. The predictive function (or mapping function) is generated in a training phase, which involves providing example inputs and ground truth (e.g. correct) outputs to the model. A test phase comprises predicting the output for a given input. Applications of machine learning include, for example, curve fitting, facial recognition and spam filtering.

In some embodiments herein, the model comprises a neural network model, such as a deep neural network model. The skilled person will be familiar with neural networks, but in brief, neural networks are a type of machine learning model that can be trained to predict a desired output for given input data. Neural networks are trained by providing training data comprising example input data and the corresponding "correct" or ground truth outcome that is desired. Neural networks comprise a plurality of layers of neurons, each neuron representing a mathematical operation that is applied to the input data. The output of each layer in the neural network is fed into the next layer to produce an output. For each piece of training data, weights associated with the neurons are adjusted (e.g. using processes such as back propagation and/or gradient decent) until the optimal weightings are found that produce predictions for the training examples that reflect the corresponding ground truths.

As noted above methods and systems herein relate to training a model such as any of the models described above, using a distributed learning process. Distributed learning processes were described above with respect to Fig. 1 and the detail therein will be understood to apply to embodiments of the apparatus 200 and the method 300. Examples of distributed learning processes include, but are not limited to Federated Learning and Distributed Data Parallelism methods.

In some embodiments the apparatus 200 may comprise a server that co-ordinates the training performed by the servers at the plurality of clinical sites, in other words, a "central server". Herein the method 300 may be performed or initiated by a user, company or any other designer or orchestrator of the training process, e.g. using the apparatus 200. Using terminology commonly associated with distributed learning schemes, the central server (e.g. such as an apparatus 200) may comprise the "master" of the scheme and the plurality of clinical sites may comprise "workers" or nodes.

The central server (e.g. apparatus 200) may store and/or maintain (e.g. update) a global model. The global model (or global copy of the model) comprises a master copy, or central copy of the model. As described in more detail below, outcomes (e.g. local updates) of the training performed at each of the plurality of clinical sites are transmitted to the central server and incorporated into the global model. Thus the global model represents the current "combined" outcome of all of the training performed at the plurality of clinical sites.

In this context a clinical site may comprise a hospital, a surgery, a clinic, and/or a datacentre or other computing site suitable for storing medical data originating from such a clinical site.

As noted above, the model is for performing a task on medical data. In this context medical data may comprise any type of data that can be used, produced and/or obtained in a medical setting, including but not limited to: clinical diagnostic data, such as patient vital signs, or physiological parameters, medical images, medical files (e.g. such as patient records), and/or outputs of medical machines (e.g. operational or diagnostic data from medical equipment).

The model may take as input one or more of the types of medical data described above and perform a task on the medical data. The task may comprise, for example, a classification task or a segmentation task. For example, the model may predict a classification for the medical data and/or provide an output classification. In embodiments herein, the model may output, for example, a patient diagnosis based on the input medical data. In embodiments where the medical data comprises a medical image, the model may output, for example, a segmentation of the medical image, a location of a feature of interest in the medical image, or a diagnosis based on the medical image. The skilled person will appreciate however that these are merely examples, and that the model may take different types of medical data as input and provide different types of outputs (e.g. perform different tasks) to the examples provided above.

Turning back to the method 300, as noted above, the method 300 comprises: a) sending (302) information to the plurality of clinical sites to enable each of the plurality of clinical sites to create a local copy of the model and train the respective local copy of the model on training data at the respective clinical site.

For example, the information may comprise model information, indicating the type of model, and/or values of parameters in the model. For example, in embodiments where the model comprises a neural network, the information may comprise parameters including, but not limited to, a number of layers in the neural network model, the input and output channels of the model, and values of the weights and biases in the neural network model. Generally the information sent in step a) is sufficient to enable each of the plurality of clinical sites to create a local copy of the model.

The information may further comprise instructions of how each clinical site is to train the model. For example, the information may indicate, for example, a number of epochs of training that is to be performed, a number of pieces of training data that should be used to train the model, the type of data that is to be used to train the model, etc.

In step b) the method 300 comprises receiving (304), from each of the plurality of clinical sites, i) a local update to a parameter in the model obtained by training the local copy of the model on the training data at the respective clinical site and ii) metadata related to a quality of the training performed at the respective clinical site.

The local update to the parameter in the model may comprise an outcome of the training of the local copy of the model on the training data at the respective clinical site. For example, a change in a parameter of the model resulting from the training. In embodiments where the model comprises a neural network, the parameter may comprise a weight or bias in the neural network, or a change that should be applied to a weight or bias in a neural network. Thus in some embodiments, the step b) comprises receiving updated values $w_i$ (or changes in values $\Delta w_i$) of one or more weights or biases in the neural network model.

The metadata is related to a quality of the training performed at the respective clinical site. In some embodiments, the metadata provides an indication of a performance of the respective local copy of the model after the training. For example, an indication of the accuracy of the local model at the respective clinical site.

In some embodiments, the metadata provides an indication of a performance of the respective local copy of the model after the training, for one or more subsets of training data having a common characteristic that is expected to influence model error. The characteristic may be expected to influence model error, for example, by making it easier (or conversely more difficult) for the model to perform the task on (e.g. classify/segment) the medical data. For example, the metadata may comprise an indication of the performance of the respective local model at classifying medical data with different quality levels, or different levels of completeness (e.g. full images compared to partial images, for example.)

In another embodiment, the metadata may comprise medical statistics that can influence the training error. Put another way, the metadata may comprise statics relating to features of the training data at the respective medical site that may influence the accuracy of the respective local model. For example, the number of training data samples of high quality compared to the number of training data samples of low quality.

The metadata provides an indication of a quality of the training data at the respective clinical site. For example, the metadata may provide an indication of a distribution of the training data at the clinical site between different output classifications of the model. In this sense, output classifications may comprise labels or categories output by the model. The metadata describes whether the training data is evenly distributed between different output classifications, or whether the training data is skewed towards particular classifications (e.g. with more training data associated with some labels compared to other labels).

For example, consider a classification problem with 5 classes (or labels), each clinical site has different ratios of data in each class and the trainable data varies as distributed learning is performed. The returned metadata may comprise the number of samples per class present in each node during weight updating. This provides an indication of how balanced the training data is (e.g. between different classes) that was used to train the respective local model. Local updates resulting from more balanced training data sets may be given more weight compared to local updates resulting from less balanced training data sets.

In step c) of the method 300, the method comprises updating (306) the parameter in the global model, based on the received local updates to the parameter and the received metadata.

Generally, the metadata is used to perform parameter merging at the central server. Therefore, the merged parameter may comprise a function of the parameters received from the clinical sites and the corresponding metadata. In other words, in some embodiments:

Merged parameter = function (metadata, parameters received from clinical sites).

Mathematically the function can be represented as follows: lets, consider n clinical sites N1, N2, N3,....., etc with parameters W1, W2, W3,.....,etc, and each of the clinical sites has a quality measure $\alpha 1$, $\alpha 2$, $\alpha 3$,...., etc, where the alpha value varies between 0 and 1 and is calculated from the metadata sent from the clinical sites to the central server. The merged parameter may thus be calculated as:

$$\text{Merged parameter} = (\alpha 1 * W1 + \alpha 2 * W2 + \alpha 3 * W3 + \ldots \ldots ) / (\alpha 1 + \alpha 2 + \alpha 3 + \ldots \ldots \ldots )$$

Put another way, in some embodiments, the step of combining the local updates to the parameter to determine the update to the global model comprises determining a parameter for the global model according to:

$$\text{Global Parameter} = (\alpha 1 * W1 + \alpha 2 * W2 + \alpha 3 * W3 + \ldots + \alpha_N * W_N) / (\alpha 1 + \alpha 2 + \alpha 3 + \ldots \alpha_N);$$

wherein $W_N$ comprises the local update to the parameter in the model as determined by the nth clinical site, and $\alpha_N$ comprises a real number in the range $0 \leq \alpha_N \leq 1$. The value of $\alpha_N$ is determined from the metadata associated with the update to the parameter in the model determined by the nth clinical site. For the avoidance of doubt, other parameters may also be used when calculating the $\alpha$ values. For example, for batch wise training, one of approach to compute $\alpha i$ comprises:

$$\alpha i = \text{number of relevant samples in ith node/ global batch size}$$

where usable samples in nth node may be obtained from metadata information of scans (slice thickness, resolution etc.) In some embodiments, step c) may comprise combining the local updates to the parameter to determine an update to the global model by weighting each local update according to the respective metadata such that local updates associated with metadata indicating high quality training outcomes have a higher weighting (e.g. a higher value of $\alpha$ as described above) compared to updates associated with metadata indicating low quality training outcomes. For example, generally, a local update associated with a more accurate local model may be given a higher weighting compared to a local update associated with a less accurate local model.

In one embodiment, the medical data comprises computed tomography, CT, scan data. In such an embodiment, the metadata may provide an indication of a performance of the respective local copy of the model at classifying CT images of different radiation dosage, e.g. the metadata may provide an indication of the performance of the model when classifying high dosage CT scans and/or (or compared to) low dosage CT scans. In such an example, the model may be expected to be able to classify CT images of high radiation dosage more accurately than CT images of low radiation dosage. In this embodiment, in step c) of the method 300, such metadata may be used to prioritise an update received from a first clinical site having a local model with higher performance on high dosage CT scans, compared to an update received from a second clinical sites having a local model with lower performance on high dosage CT scans, e.g. even if the performance of the first model on low dosage CT scans is comparatively poor.

In another embodiment, the metadata may describe the number of training data samples that are low dose or high dose for contrast enhancement. As noted above, if a model makes mistakes on low does CT image this error is given less weightage when compared to a model making a mistake on high dose CT (As the expectation is for the algorithm to perform very well on high dose CT images and a few mistakes on low dose CT images will be acceptable).

In another example, the metadata may comprise an indication of the performance of the model when classifying training data of different completeness levels. For example, in an embodiment where the model is trained to perform a segmentation of an anatomical feature in medical imaging data; and wherein the metadata comprises an indication of the performance of the model when segmenting full images of the anatomical feature and/or partial images of the anatomical feature. In this embodiment, in step c) of the method 300, such metadata may be used to prioritise an update received from a first clinical site having a local model with higher performance when segmenting full images of the anatomical feature, compared to an update received from a second clinical sites having a local model with lower performance when segmenting full images of the anatomical feature, e.g. even if the performance of the first model on when segmenting partial images of the anatomical feature is comparatively poor.

In an embodiment where the medical data comprises CT scan data and the model is for segmentation of the Liver in the CT scan data, the metadata may comprise, for example, the following information:

1. Error on Low Dose CT and Error on High dose CT
2. Error based on Area of segmentation

When observing a CT volume, all of the liver will not be visible on every slice of the CT volume, if the model is making a mistake when the liver is partially visible, this error should be acceptable and must have a smaller error when compared to mistakes being made on image slices when the whole liver is visible, i.e. during distributed learning of liver segmentation, on one node the mistakes are happening where the liver partially visible and on the second node the mistakes are happening during both scenarios where liver is partially visible and liver is completely visible the algorithm should give weightage to the update coming from the former node.

Thus, in the manner described above, a global model may be updated using metadata that provides a deeper insight into the quality of local updates determined by a plurality of clinical sites in a distributed learning scheme. As described above, where training data originates from different clinical sites, there is the potential for bias and/or model drift to influence the model. The method herein proves means to reduce this and combat model bias, and data heterogeneity.

Turning now to other embodiments, in some embodiments herein, the method 300 may be improved further by detecting whether the global model is drifting during the training process, based on the analysis of the visualization output. For example, if the region of interest that is activated/considered by the model when determining a classification or label keeps varying, the drift associated can be ascertained. The variation value may be computed based on benchmark training data, that is fed through the global model at different time points during the training process. (e.g. at time point t0 and the change in the variation is obtained at t1). The variation computation may be in terms of the coordinate value, or area under the bounding box of the region of interest that is activated/considered by the model.

For example, model drift may be determined according to:

$$\text{Model Drift}: |(\text{coordinate at t0}) - (\text{coordinate at t1})) > \text{Dynamic threshold}$$

In other words, preceding steps a), b) and c) (e.g. at a time t0), the method 300 may comprise determining, for a test medical image, a first region of the test image used by the global model to perform the task on the test medical image. The method may then further comprise, following steps a), b) and c): determining, for the test medical image, a second region of the test image used by the updated global model to perform the task on the test medical image, and comparing the first region of the test image to the second region of the test image to determine a measure of model drift.

The step of comparing may comprise, for example, comparing co-ordinates associated with the first and second regions (e.g. at the centre or edges of the regions or bounding boxes), or comparing the areas within the first and second regions and determining whether the regions have changed e.g. by a statistically significant amount, or greater than a threshold amount.

In this context, the dynamic threshold may be determined based on the current content and the different models and also the type of the model under question. Hence it is not static for all applications/model types.

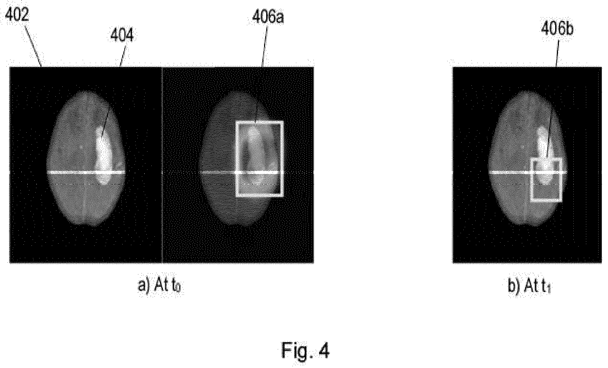

This is illustrated in Fig. 4 which shows an image of a liver 402 comprising a lesion 404. A model is used to classify (e.g. locate) the lesion. The model is trained according to the method 300 above. Preceding the steps a), b) and c), at a time $t_0$, the model classifies the lesion based on the region of the image 406a. At a time $t_1$, the model classifies the same lesion based on the region of the image 406b. The difference in the locations and size of the regions 406a and 406b may indicate that the model has drifted. Thus, by comparing and monitoring changes in the region between different training epochs/updates, drift of the model may be determined.

In other embodiments, the steps a), b) and c) may be repeated, e.g. to provide a sequence of training epochs. For example, steps a), b) and c) may be repeated periodically, or each time new training data becomes available at a clinical site.

Turning to other embodiments, in some embodiments, the method may be augmented though the use of Active Learning. The skilled person will be familiar with active learning, but in brief in active learning focusses training on training data that has previously been miss-classified or classified with a low probability of accuracy by the model. Thus effectively focussing the training on areas of weakness in the model.

In some embodiments, the method may thus comprise repeating steps a), b) and c) for a subset of the training data at each respective clinical site that was classified by the model with a certainty below a threshold certainty level. For example, the certainty may be measured using confidence levels output by the model. In other embodiments, the certainty with which the model classified the data may be calculated using a measure of entropy. Measures of entropy may reflect an amount of information in a dataset. Thus, the higher the entropy, the higher the amount of information in the dataset. Thus, for example, if a dataset has high entropy, it has variety in its content.

Generally, an ambiguity zone may be defined comprising training data for which the classification is uncertain. Training data in such an ambiguity zone may be used in subsequent epochs of model training. It is noted that the ambiguity zone may be dynamic, and change between epochs as the (global) model improves.

Furthermore, in these embodiments, where optimized (e.g. active) distributed learning is performed, in which the model is trained on misclassified training samples each time, the metadata and thus, the quality measure value ($\alpha$) as described above, may change for each training epoch.

In this manner, the most relevant training data is considered in each training epoch which adds more value to the model. In this way "optimized" distributed learning, which considers only misclassified examples for updating the weights in a subsequent epoch may be performed. The proposed concept captures variation in the data set whilst making sure that data does not leave hospital premises. Also, the new concepts designed make sure to train model that gives high performance with less data.

One issue with distributed learning processes in general is that as the predictions of the model improve, the parameter updates become smaller (by applying a simple averaging or weighted averaging the weight updates gets negated). This can, at times, cause a distributed learning model to not perform as well as the centralized model. The use of active learning as described above helps to overcome this problem, by considering only the misclassified (properties of Active Learning) or images without proper segmentation or classification for re-training. There are various advantages of this: Each time the training data reduces which results in faster training time; Since the algorithm only trains on the misclassified data, the loss function is more focused and gradients used for updating the model may be better.

Generally, assuming the data from all clinical sites will be very similar is a utopian idea. Therefore considering the quality of misclassified data (according to the active learning principle) as metadata and using this information while merging the weights will help build a better global model.

The idea of combining distributed learning and active learning follows a philosophy of "think global" and "act local". Global models can be taught using training data from different hospitals located globally through distributed learning, with improved model performance at individual nodes through active learning. Distributed learning captures the variation of data across human populations located (potentially) globally, whereas active learning improves the performance at local nodes with lesser data.

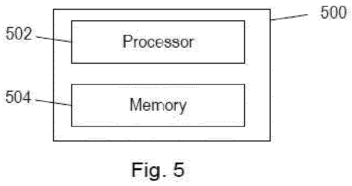

Turning now to the perspective of the clinical sites that perform the local training as described above, Fig. 5 illustrates an apparatus 500 for use in a clinical site for training a model to perform a task on medical data using a distributed machine learning process, according to some embodiments herein. Generally, the apparatus may form part of a computer apparatus or system e.g. such as a laptop, desktop computer or other computing device. In some embodiments, the apparatus 500 may form part of a distributed computing arrangement or the cloud.

The apparatus comprises a memory 504 comprising instruction data representing a set of instructions and a processor 502 (e.g. processing circuitry or logic) configured to communicate with the memory and to execute the set of instructions. Generally, the set of instructions, when executed by the processor, may cause the processor to perform any of the embodiments of the method 600 as described below.

Embodiments of the apparatus 500 may be for use in a clinical site for training a model to perform a task on medical data using a distributed machine learning process whereby a global model at a central server is updated based on training performed on a local copy of the model at the clinical site. More specifically, the set of instructions, when executed by the processor, cause the processor to: receive information from a central server enabling a local copy of the model to be created and trained on training data at the clinical site; train a local copy of the model according to the information; and send to the central server, i) an update to the model based on training of the local copy of the model on the training data at the clinical site and ii) metadata related to a quality of the training performed at the respective clinical site.

The processor 502 can comprise one or more processors, processing units, multi-core processors or modules that are configured or programmed to control the apparatus 500 in the manner described herein. In particular implementations, the processor 502 can comprise a plurality of software and/or hardware modules that are each configured to perform, or are for performing, individual or multiple steps of the method described herein. The processor 502 can comprise one or more processors, processing units, multi-core processors and/or modules that are configured or programmed to control the apparatus 500 in the manner described herein. In some implementations, for example, the processor 502 may comprise a plurality of (for example, interoperated) processors, processing units, multi-core processors and/or modules configured for distributed processing. It will be appreciated by a person skilled in the art that such processors, processing units, multi-core processors and/or modules may be located in different locations and may perform different steps and/or different parts of a single step of the method described herein.

The memory 504 is configured to store program code that can be executed by the processor 502 to perform the method described herein. Alternatively or in addition, one or more memories 504 may be external to (i.e. separate to or remote from) the apparatus 500. For example, one or more memories 504 may be part of another device. Memory 504 can be used to store the local copy of the model, the training data, the outputs of the training and/or any other information or data received, calculated or determined by the processor 502 of the apparatus 500 or from any interfaces, memories or devices that are external to the apparatus 500. The processor 502 may be configured to control the memory 504 to store the local copy of the model, the training data, the outputs of the training and/or any other information or data produced by or used during the method 600 described below.

In some embodiments, the memory 504 may comprise a plurality of sub-memories, each sub-memory being capable of storing a piece of instruction data. For example, at least one sub-memory may store instruction data representing at least one instruction of the set of instructions, while at least one other sub-memory may store instruction data representing at least one other instruction of the set of instructions.

It will be appreciated that Fig. 5 only shows the components required to illustrate this aspect of the disclosure and, in a practical implementation, the apparatus 500 may comprise additional components to those shown. For example, the apparatus 500 may further comprise a display. A display may comprise, for example, a computer screen, and/or a screen on a mobile phone or tablet. The apparatus may further comprise a user input, such as a keyboard, mouse or other input device that enables a user to interact with the apparatus, for example, to provide initial input parameters to be used in the method described herein. The apparatus 500 may comprise a battery or other power supply for powering the apparatus 500 or means for connecting the apparatus 500 to a mains power supply.

Turning to Fig. 600, there is a computer implemented method 600 for use in training a model to perform a task on medical data using a distributed machine learning process whereby a global model at a central server is updated based on training performed on a local copy of the model at the clinical site. Embodiments of the method 600 may be performed, for example by an apparatus such as the apparatus 500 described above.

Briefly, in a first step 602, the method 600 comprises: receiving information from a central server enabling a local copy of the model to be created and trained on training data at the clinical site. In a second step 604, the method comprises training a local copy of the model according to the information. In a third step 606 the method comprises sending to the central server, i) an update to the model based on training of the local copy of the model on the training data at the clinical site and ii) metadata related to a quality of the training performed at the respective clinical site.

The corresponding method and apparatus to the central server were described above with respect to Figs. 2 and 3 and the detail therein will be understood to apply equally to the method in the clinical site.

In this context a clinical site 500 may comprise a server (e.g. a "clinical server") or a datacentre associated with a hospital, a surgery, a clinic, or any other medical facility. A clinical site may comprise, for example, a datacentre such as a Hospital Data Centre (HDC) or any other computing site suitable for storing medical data.

The information received in step 602 was described above with respect to Figs. 2 and 3 and the detail therein will be understood to apply equally to the apparatus 500 and the method 600. Using the information, the clinical site creates a local copy of the model and trains the local copy of the model using training data at the clinical site (e.g. according to the information received from the central server).

The skilled person will be familiar with methods of training a machine learning model, for example using methods including but not limited to gradient descent, and back-propagation.

The clinical site obtains metadata related to a quality of the training performed at the respective clinical site on the local model, and in step 606, sends i) an update to the model based on training of the local copy of the model on the training data at the clinical site (e.g. the outcome of the training) and ii) the metadata to the central server. The metadata was described in detail above with respect to the apparatus 200 and the method 300 and the details therein will be understood to apply equally to the apparatus 500 and the method 600.

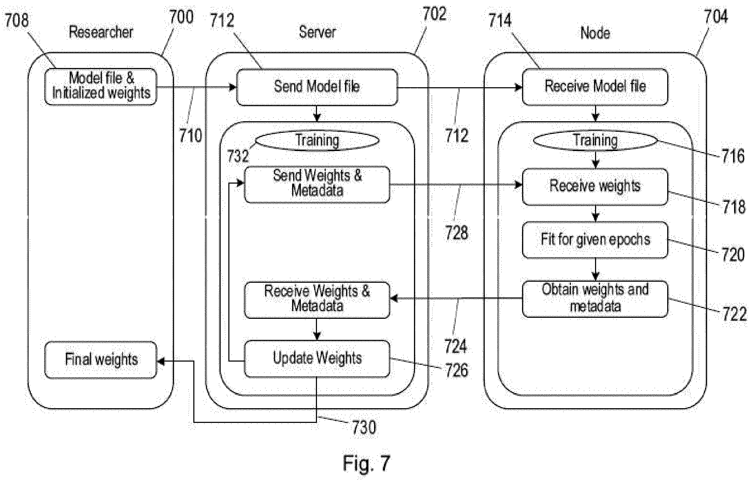

Turning now to another embodiment, Fig. 7 illustrates a method of training a model using a distributed learning process according to some embodiments herein. In this embodiment there is a Researcher or other user on a computer or server 700, a central server 702 and a plurality of clinical sites (or nodes) 704. Only one clinical site 704 is shown in Fig. 7 for clarity. In this embodiment, the model comprises a neural network. The method is as follows.

The researcher develops the model and places it on a server along with the pre initialized weights 708. The following process is then performed:

710. Researcher sends the model and initialized weights to the server 702. This starts the server. The Server waits for the nodes 704 to connect

712. Once server connects the deep learning model is passed to the node, the connection between the server and the node is encrypted. The model is received 714 by the node 704.

716. The node creates a local copy of the model and performs training on the local copy of the model. Training proceeds using an Active Learning method, whereby the initialized model is used to perform predictions (or classifications) on training data at the node. If a prediction has a confidence less than a certain threshold confidence value (the threshold confidence value is assigned by the researcher e.g. dice score less than 0.95), then this is used for further training of the model. The model is trained for a number of epochs, mentioned in the model file. Generally, the training includes receiving weight values 718, fitting for different epochs 720 and obtaining final weights and metadata 722.

724. Weights are returned to the central server 702 along with metadata related to the quality of the training performed.

726. The returned weights are merged with the help of the metadata returned from the nodes using averaging or weighted averaging or other statistical methods seen fit by the researcher and used to update the global model (e.g. the version of the model stored on the central server 602). Information describing the updated global model is then sent back 726 to the node 704 for retraining with the new merged weights.

This process is performed in an iterative manner till the model converges. The transfers between the central server 704 and node 706 may be logged in a database (this can also be recorded on a block chain, so that the records cannot be deleted). This step may be used to maintain privacy.

Once the model has converged, the final weights may be sent 730 to the researcher.

It is noted that the central server may also perform training 732 on its own local copy of the model, if the central server has local training data stored thereon (e.g. in some embodiments, the central server may comprise a server at a clinical site, that trains its own local copy of the model whilst also co-ordinating the distributed training process amongst the plurality of clinical sites.)

Turning now to another embodiment, in some embodiments, there is the use of a model trained according to any of the methods or apparatus described herein (e.g. the methods 300, 600 or 700 or the apparatus 200 or 500) to perform a task on medical data. The use may be performed in addition to, or separately from the methods described herein. Examples of use include but are not limited to, for example, segmenting an image (such as CT scan of a liver) using a model trained according to any of the methods herein; classifying (e.g. diagnosing or making some other classification of) medical records using a model trained according to any of the methods herein.

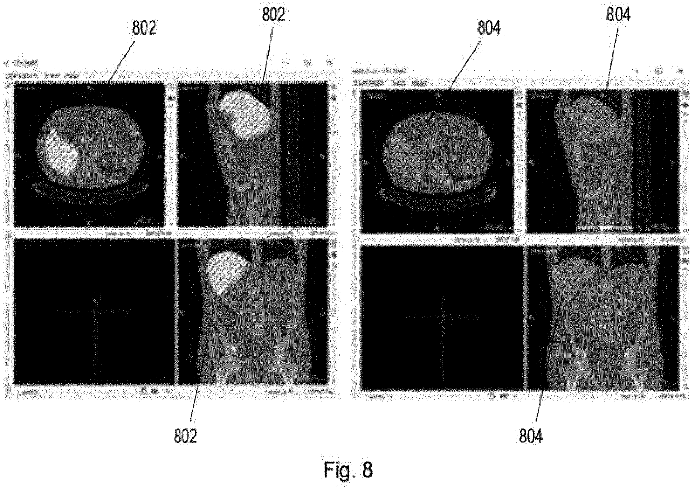

Turning now to Fig. 8 which shows an output segmentation of a liver 802 produced by a model trained using a traditional distributed learning process compared to a segmentation of a liver 804 output by a model trained using the methods 300 and 600 described above.

In another embodiment, there is provided a computer program product comprising a computer readable medium, the computer readable medium having computer readable code embodied therein, the computer readable code being configured such that, on execution by a suitable computer or processor, the computer or processor is caused to perform the method or methods described herein.

Thus, it will be appreciated that the disclosure also applies to computer programs, particularly computer programs on or in a carrier, adapted to put embodiments into practice. The program may be in the form of a source code, an object code, a code intermediate source and an object code such as in a partially compiled form, or in any other form suitable for use in the implementation of the method according to the embodiments described herein.

It will also be appreciated that such a program may have many different architectural designs. For example, a program code implementing the functionality of the method or system may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

Appendix 1

Experimental Data

Experiment 1: Sample size: number of samples at each node (Class imbalance)

Model Type: Neural Network trained on the Modified National Institute of Standards and Technology database (MINST). MINST contains handwritten images of the numerals 0 to 9. The model is trained to classify each image based on its numerical content.

In an experiment, medical data was available at 2 nodes. The training dataset had 10 different classes. In the first node, we have 9 classes of similar prevalence and a small number of samples from class 10. At the second node, the first 9 classes of data are very sparse and the 10th class of data is prevalent.

In above example, models were merged using two approaches,

> 1) simple average merging : models were merged without metadata information. Merged model gives 20% accuracy.
> 2) Weighted average merging : We merge models, using metadata information, (class prevalence). This model has achieved 90% and 88.9% accuracy with train and test data respectively.

Results are summarised below.

|  | Node-1 | Node 2 | Merged (simple average) | Merged (weighted average) |
|---|---|---|---|---|
| Train Accuracy | 0.8971 | 0.09915 | 0.19358333 | 0.90055 |
| Test Accuracy | 0.886500 | 0.100900 | 0.200300 | 0.889700 |

Experiment 2: Acquisitions/image scanner settings

Model: Neural Network

Assume, we have data available at 2 nodes. Dataset at node 1 and 2 are acquired using different CT machines at two different locations.

In above example, data is acquired from two different locations. First dataset has mean HU intensity of 60 HU, where second dataset has mean HU intensity of 100. To perform federated learning, it will require use of advance pre-processing techniques, based on metadata. For example, if the mean intensity of one site is slightly different than the expected values, the data can still be used, but with a lower weighting for that site (for example, scaled based on degree or difference from the expected values.) That is, we indicate that the weight updates from this site should be given lower priority as the distribution is not as expected. If the data is completely on a different scale the weight can be made zero so that it does not spoil the model completely. Generally, if the data from two sites are different or have different properties then the model is more likely to fail. The weights assigned to a site can thus be varied based on the statistical heterogeneity to find the best model from the given sites. This improves the accuracy of the resulting global model.

Experiment 3: Image quality (CT scans)

Model: Neural Network

Assume, we have data available at 2 nodes. Dataset at node 1 and 2 are acquired using different CT machines at two different locations. The quality of data acquired from two centers are completely different. Based on intensity histogram compression, we can perform, weighted merge. Where higher weights are assigned based on similarity to the overall sample dataset.

This allows the model not to be penalized where mistakes are made with the low-quality data by assigning lower weights. This improves the accuracy of the resulting global model.

## Claims

1. A computer implemented method of training a model to perform a task on medical data using a distributed machine learning process whereby a global model is updated based on training performed on local copies of the model at a plurality of clinical sites, wherein the model is for use in predicting a classification for the medical data, wherein the medical data comprises a medical image, the method comprising:

   a) sending (302) information to the plurality of clinical sites to enable each of the plurality of clinical sites to create a local copy of the model and train the respective local copy of the model on training data at the respective clinical site;
   b) receiving (304), from each of the plurality of clinical sites, i) a local update to a parameter in the model obtained by training the local copy of the model on the training data at the respective clinical site and ii) metadata providing an indication of a quality of the training data at the respective clinical site by providing an indication of how balanced the training data at the clinical site is between different output classifications of the model; and
   c) updating (306) the parameter in the global model, based on the received local updates to the parameter and the received metadata by combining the local updates to the parameter to determine an update to the global model by weighting each local update according to the respective metadata such that local updates resulting from more balanced training data sets are given more weight compared to local updates resulting from less balanced training data sets.

2. A method as in claim 1 wherein the step of combining the local updates to the parameter to determine the update to the global model comprises:

   determining a parameter for the global model according to:

   $$\text{Global Parameter} = (\alpha1 * W1 + \alpha2 * W2 + \alpha3 * W3 + \ldots + \alpha_N * W_N) / (\alpha1 + \alpha2 + \alpha3 + \ldots \alpha_N);$$

   wherein $W_N$ comprises the local update to the parameter in the model as determined by the nth clinical site, and $\alpha_N$ comprises a real number in the range $0 \leq \alpha_N \leq 1$; and
   wherein the value of $\alpha_N$ is determined from the metadata associated with the update to the parameter in the model determined by the nth clinical site.

3. A method as in any one of the preceding claims, wherein the metadata comprises the number of samples per class present at the clinical site during weight updating.

4. A method as in any one of the preceding claims wherein the medical data comprises a medical image, the method further comprising:

preceding steps a), b) and c):
determining, for a test medical image, a first region of the test image used by the global model to perform the task on the test medical image; and
following steps a), b) and c):

determining, for the test medical image, a second region of the test image used by the updated global model to perform the task on the test medical image; and
comparing the first region of the test image to the second region of the test image to determine a measure of model drift.

5. A method as in any one of the preceding claims further comprising:
repeating steps a), b) and c) for a subset of the training data at each respective clinical site that was classified by the model with a certainty below a threshold certainty level.

6. A computer implemented method at a clinical site for training a model to perform a task on medical data using a distributed machine learning process whereby a global model at a central server is updated based on training performed on a local copy of the model at the clinical site and metadata, wherein the model is for use in predicting a classification for the medical data, wherein the medical data comprises a medical image, the method comprising:

receiving information from a central server enabling a local copy of the model to be created and trained on training data at the clinical site;
training a local copy of the model according to the information; and
sending to the central server, i) an update to the model based on training of the local copy of the model on the training data at the clinical site and ii) the metadata providing an indication of a quality of the training data at the respective clinical site by providing an indication of how balanced the training data at the clinical site is between different output classifications of the model.

7. A method as in claim 6, wherein the metadata comprises the number of samples per class present at the clinical site during weight updating.

8. A method as in any one of the preceding claims wherein the model comprises a neural network model and the parameter comprises a weight or a bias in the neural network model.

9. An apparatus for training a model to perform a task on medical data using a distributed machine learning process whereby a global model is updated based on training performed on local copies of the model at a plurality of clinical sites, wherein the model is for use in predicting a classification for the medical data, wherein the medical data comprises a medical image, the apparatus comprising:

a memory comprising instruction data representing a set of instructions; and
a processor configured to communicate with the memory and to execute the set of instructions, wherein the set of instructions, when executed by the processor, cause the processor to:

a) send information to the plurality of clinical sites to enable each of the plurality of clinical sites to create a local copy of the model and train the respective local copy of the model on training data at the respective clinical site;
b) receive, from each of the plurality of clinical sites, i) a local update to a parameter in the model obtained by training the local copy of the model on the training data at the respective clinical site and ii) metadata providing an indication of a quality of the training data at the respective clinical site by providing an indication of how balanced the training data at the clinical site is between different output classifications of the model; and
c) update the parameter in the global model, based on the received local updates to the parameter and the received metadata by combining the local updates to the parameter to determine an update to the global model by weighting each local update according to the respective metadata such that local updates resulting from more balanced training data sets are given more weight compared to local updates resulting from less balanced training data sets.

10. A computer program product comprising a computer readable medium, the computer readable medium having computer readable code embodied therein, the computer readable code being configured such that, on execution by a suitable computer or processor, the computer or processor is caused to perform the method as claimed in any one of claims 1 to 8.

**Patentansprüche**

1. Computerimplementiertes Verfahren zum Trainieren eines Modells, um eine Aufgabe auf medizinischen Daten unter Verwendung eines verteilten maschinellen Lernprozesses durchzuführen, wodurch ein globales Modell basierend auf Training aktualisiert wird, das an lokalen Kopien des Modells an einer Vielzahl von klinischen Standorten durchgeführt wird, wobei das Modell zur Verwendung beim Vorhersagen einer Klassifizierung für die medizinischen Daten dient, wobei die medizinischen Daten ein medizinisches Bild umfassen, wobei das Verfahren umfasst:

a) Senden (302) von Informationen an die Vielzahl von klinischen Standorten, um jedem der Vielzahl von klinischen Standorten zu ermöglichen, eine lokale Kopie des Modells zu erstellen und die jeweilige lokale Kopie des Modells auf Trainingsdaten am jeweiligen klinischen Standort zu trainieren;

b) Empfangen (304), von jedem der Vielzahl von klinischen Standorten i), einer lokalen Aktualisierung eines Parameters in dem Modell, die durch Trainieren der lokalen Kopie des Modells auf den Trainingsdaten am jeweiligen klinischen Standort erhalten wird, und ii) Metadaten, die einen Hinweis auf eine Qualität der Trainingsdaten am jeweiligen klinischen Standort durch Bereitstellen eines Hinweises darauf bereitstellen, wie ausgewogen die Trainingsdaten am klinischen Standort zwischen verschiedenen Ausgabeklassifizierungen des Modells sind; und

c) Aktualisieren (306) des Parameters im globalen Modell basierend auf den empfangenen lokalen Aktualisierungen des Parameters und den empfangenen Metadaten durch Kombinieren der lokalen Aktualisierungen des Parameters, um eine Aktualisierung des globalen Modells durch Gewichten jeder lokalen Aktualisierung gemäß den jeweiligen Metadaten zu bestimmen, sodass lokalen Aktualisierungen, die sich aus ausgewogeneren Trainingsdatensätzen ergeben, verglichen mit lokalen Aktualisierungen, die sich aus weniger ausgewogenen Trainingsdatensätzen ergeben, mehr Gewicht beigemessen wird.

2. Verfahren nach Anspruch 1, wobei der Schritt des Kombinierens der lokalen Aktualisierungen des Parameters, um die Aktualisierung des globalen Modells zu bestimmen, umfasst:
Bestimmen eines Parameters für das globale Modell gemäß:

$$\text{Globale Parameter} = (\alpha 1 * W1 + \alpha 2 * W2 + \alpha 3 * W3 + ....+ \alpha_N * W_N)/(\alpha 1 + \alpha 2 + \alpha 3 .... \alpha_N);$$

wobei $W_N$ die lokale Aktualisierung des Parameters in dem Modell, wie durch den n-ten klinischen Standort bestimmt, umfasst, und $\alpha_N$ eine reelle Zahl im Bereich $0 \leq \alpha_N \leq 1$ umfasst; und
wobei der Wert von $\alpha_N$ aus den Metadaten bestimmt wird, die mit der Aktualisierung des Parameters in dem Modell verknüpft sind, der vom n-ten klinischen Standort bestimmt wird.

3. Verfahren nach einem der vorstehenden Ansprüche, wobei die Metadaten die Anzahl der Proben je Klasse umfassen, die während der Gewichtungsaktualisierung am klinischen Standort vorhanden sind.

4. Verfahren nach einem der vorstehenden Ansprüche, wobei die medizinischen Daten ein medizinisches Bild umfassen, wobei das Verfahren weiter umfasst:

vor den Schritten a), b) und c):
Bestimmen, für ein medizinisches Testbild, eines ersten Bereichs des Testbilds, der von dem globalen Modell verwendet wird, um die Aufgabe am medizinischen Testbild durchzuführen; und
anschließend an Schritte a), b) und c):

Bestimmen für das medizinische Testbild eines zweiten Bereichs des Testbilds, der vom aktualisierten globalen Modell verwendet wird, um die Aufgabe am medizinischen Testbild durchzuführen; und

Vergleichen des ersten Bereichs des Testbilds mit dem zweiten Bereich des Testbilds, um ein Maß einer Modelldrift zu bestimmen.

**5.** Verfahren nach einem der vorstehenden Ansprüche, weiter umfassend:
Wiederholen von Schritten a), b) und c) für eine Teilmenge der Trainingsdaten an jedem jeweiligen klinischen Standort, die von dem Modell mit einer Gewissheit unterhalb eines Gewissheitsschwellenwerts klassifiziert werden.

**6.** Computerimplementiertes Verfahren an einem klinischen Standort zum Trainieren eines Modells, um eine Aufgabe auf medizinischen Daten unter Verwendung eines verteilten maschinellen Lernprozesses durchzuführen, wodurch ein globales Modell auf einem zentralen Server basierend auf Training aktualisiert wird, das an einer lokalen Kopie des Modells am klinischen Standort und Metadaten durchgeführt wird, wobei das Modell zur Verwendung beim Vorhersagen einer Klassifizierung für die medizinischen Daten dient, wobei die medizinischen Daten ein medizinisches Bild umfassen, wobei das Verfahren umfasst:

Empfangen von Informationen von einem zentralen Server, wodurch ermöglicht wird, dass eine lokale Kopie des Modells am klinischen Standort erstellt, und anhand von Trainingsdaten trainiert wird;
Trainieren einer lokalen Kopie des Modells gemäß den Informationen; und
Senden an den zentralen Server i) einer Aktualisierung des Modells basierend auf Training der lokalen Kopie des Modells auf den Trainingsdaten am klinischen Standort und ii) der Metadaten, die einen Hinweis auf eine Qualität der Trainingsdaten am jeweiligen klinischen Standort durch Bereitstellen eines Hinweises darauf bereitstellen, wie ausgewogen die Trainingsdaten am klinischen Standort zwischen verschiedenen Ausgabeklassifizierungen des Modells sind.

**7.** Verfahren nach Anspruch 6, wobei die Metadaten die Anzahl der Proben je Klasse umfassen, die während der Gewichtungsaktualisierung am klinischen Standort vorhanden sind.

**8.** Verfahren nach einem der vorstehenden Ansprüche, wobei das Modell ein neuronales Netzwerkmodell umfasst und der Parameter eine Gewichtung oder eine Vorspannung im neuronalen Netzwerkmodell umfasst.

**9.** Einrichtung zum Trainieren eines Modells, um eine Aufgabe auf medizinischen Daten unter Verwendung eines verteilten maschinellen Lernprozesses durchzuführen, wodurch ein globales Modell basierend auf Training aktualisiert wird, das an lokalen Kopien des Modells an einer Vielzahl von klinischen Standorten durchgeführt wird, wobei das Modell zur Verwendung beim Vorhersagen einer Klassifizierung für die medizinischen Daten dient, wobei die medizinischen Daten ein medizinisches Bild umfassen, wobei die Einrichtung umfasst:

einen Speicher, der Anweisungsdaten umfasst, die einen Satz von Anweisungen darstellen; und
einen Prozessor, der konfiguriert ist, um mit dem Speicher zu kommunizieren und den Satz von Anweisungen auszuführen, wobei der Satz von Anweisungen, wenn er vom Prozessor ausgeführt wird, den Prozessor veranlasst:

a) Informationen an die Vielzahl von klinischen Standorten zu senden, um jedem der Vielzahl von klinischen Standorten zu ermöglichen, eine lokale Kopie des Modells zu erstellen und die jeweilige lokale Kopie des Modells auf Trainingsdaten am jeweiligen klinischen Standort zu trainieren;
b) von jedem der Vielzahl von klinischen Standorten i) eine lokale Aktualisierung eines Parameters in dem Modell, die durch Trainieren der lokalen Kopie des Modells auf den Trainingsdaten am jeweiligen klinischen Standort erhalten wird, und ii) Metadaten, die einen Hinweis auf eine Qualität der Trainingsdaten am jeweiligen klinischen Standort durch Bereitstellen eines Hinweises darauf bereitstellen, wie ausgewogen die Trainingsdaten am klinischen Standort zwischen verschiedenen Ausgabeklassifizierungen des Modells sind, zu empfangen; und
c) den Parameter im globalen Modell basierend auf den empfangenen lokalen Aktualisierungen des Parameters und den empfangenen Metadaten durch Kombinieren der lokalen Aktualisierungen des Parameters zu aktualisieren, um eine Aktualisierung des globalen Modells durch Gewichten jeder lokalen Aktualisierung gemäß den jeweiligen Metadaten zu bestimmen, sodass lokalen Aktualisierungen, die sich aus ausgewogeneren Trainingsdatensätzen ergeben, verglichen mit lokalen Aktualisierungen, die sich aus weniger ausgewogenen Trainingsdatensätzen ergeben, mehr Gewicht beigemessen wird.

**10.** Computerprogrammprodukt, das ein computerlesbares Medium umfasst, wobei das computerlesbare Medium einen darin eingebetteten computerlesbaren Code aufweist, wobei der computerlesbare Code konfiguriert ist, sodass, bei Ausführung durch einen geeigneten Computer oder Prozessor, der Computer oder Prozessor veranlasst wird, das Verfahren nach einem der Ansprüche 1 bis 8 durchzuführen.

## Revendications

1. Procédé mis en œuvre par ordinateur pour former un modèle à effectuer une tâche sur des données médicales à l'aide d'un processus d'apprentissage automatique distribué, selon lequel un modèle global est mis à jour sur la base d'une formation effectuée sur des copies locales du modèle au niveau d'une pluralité de sites cliniques, dans lequel le modèle est destiné à être utilisé pour prédire une classification des données médicales, dans lequel les données médicales comprennent une image médicale, le procédé comprenant :

    a) l'envoi (302) d'informations à la pluralité de sites cliniques pour permettre à chacun de la pluralité de sites cliniques de créer une copie locale du modèle et de former la copie locale respective du modèle sur des données de formation au site clinique respectif ;
    b) la réception (304), de chacun de la pluralité de sites cliniques, i) d'une mise à jour locale d'un paramètre dans le modèle obtenu en formant la copie locale du modèle sur les données de formation au site clinique respectif et ii) de métadonnées fournissant une indication d'une qualité des données de formation au site clinique respectif en fournissant une indication de la manière dont les données de formation sont équilibrées au site clinique entre différentes classifications de sortie du modèle ; et
    c) la mise à jour (306) du paramètre dans le modèle global, sur la base des mises à jour locales reçues du paramètre et des métadonnées reçues, en combinant les mises à jour locales du paramètre pour déterminer une mise à jour du modèle global en pondérant chaque mise à jour locale en fonction des métadonnées respectives, de telle sorte que des mises à jour locales résultant d'ensembles de données de formation plus équilibrés reçoivent plus de poids par comparaison à des mises à jour locales résultant d'ensembles de données de formation moins équilibrés.

2. Procédé selon la revendication 1, dans lequel l'étape de combinaison des mises à jour locales du paramètre pour déterminer la mise à jour du modèle global comprend :
    la détermination d'un paramètre pour le modèle global selon :

$$\text{Paramètre global} = (\alpha 1 * W1 + \alpha 2 * W2 + \alpha 3 * W3 + ....+ \alpha_N * W_N) / (\alpha 1 + \alpha 2 + \alpha 3 .... \alpha_N) ;$$

    dans lequel $W_N$ comprend la mise à jour locale du paramètre dans le modèle tel que déterminé par le n-ième site clinique, et $\alpha_N$ comprend un nombre réel dans la plage $0 \leq \alpha_N \leq 1$ ; et
    dans lequel la valeur de $\alpha_N$ est déterminée à partir des métadonnées associées à la mise à jour du paramètre dans le modèle déterminé par le n-ième site clinique.

3. Procédé selon l'une quelconque des revendications précédentes, dans lequel les métadonnées comprennent le nombre d'échantillons par classe présents au site clinique pendant une mise à jour de poids.

4. Procédé selon l'une quelconque des revendications précédentes, dans lequel les données médicales comprennent une image médicale, le procédé comprenant en outre :

    avant les étapes a), b) et c) :
    la détermination, pour une image médicale de test, d'une première région de l'image de test utilisée par le modèle global pour effectuer la tâche sur l'image médicale de test ; et
    après les étapes a), b) et c) :

    la détermination, pour l'image médicale de test, d'une seconde région de l'image de test utilisée par le modèle global mis à jour pour effectuer la tâche sur l'image médicale de test ; et
    la comparaison de la première région de l'image de test à la seconde région de l'image de test pour déterminer une mesure de dérive de modèle.

5. Procédé selon l'une quelconque des revendications précédentes, comprenant en outre :
    la répétition des étapes a), b) et c) pour un sous-ensemble des données de formation au niveau de chaque site clinique respectif qui a été classé par le modèle avec une certitude inférieure à un niveau de certitude seuil.

6. Procédé mis en œuvre par ordinateur au niveau d'un site clinique pour former un modèle à effectuer une tâche sur des données médicales à l'aide d'un processus d'apprentissage automatique distribué, selon lequel un modèle global au niveau d'un serveur central est mis à jour sur la base d'une formation effectuée sur une copie locale du modèle au site clinique et des métadonnées, dans lequel le modèle est destiné à être utilisé pour prédire une classification des données médicales, dans lequel les données médicales comprennent une image médicale, le procédé comprenant :

la réception d'informations en provenance d'un serveur central permettant de créer une copie locale du modèle et de le former sur des données de formation au site clinique ;
la formation d'une copie locale du modèle en fonction des informations ; et
l'envoi au serveur central, i) d'une mise à jour du modèle sur la base d'une formation de la copie locale du modèle sur les données de formation au site clinique et ii) des métadonnées fournissant une indication d'une qualité des données de formation au site clinique respectif en fournissant une indication de la manière dont les données de formation sont équilibrées au site clinique entre différentes classifications de sortie du modèle.

7. Procédé selon la revendication 6, dans lequel les métadonnées comprennent le nombre d'échantillons par classe présents au site clinique pendant une mise à jour de poids.

8. Procédé selon l'une quelconque des revendications précédentes, dans lequel le modèle comprend un modèle de réseau neuronal et le paramètre comprend un poids ou un biais dans le modèle de réseau neuronal.

9. Appareil pour former un modèle à effectuer une tâche sur des données médicales à l'aide d'un processus d'apprentissage automatique distribué, selon lequel un modèle global est mis à jour sur la base d'une formation effectuée sur des copies locales du modèle au niveau d'une pluralité de sites cliniques, dans lequel le modèle est destiné à être utilisé pour prédire une classification des données médicales, dans lequel les données médicales comprennent une image médicale, l'appareil comprenant :

une mémoire comprenant des données d'instruction représentant un ensemble d'instructions ; et
un processeur configuré pour communiquer avec la mémoire et pour exécuter l'ensemble d'instructions, dans lequel l'ensemble d'instructions, lorsqu'il est exécuté par le processeur, amène le processeur à :

a) envoyer des informations à la pluralité de sites cliniques pour permettre à chacun de la pluralité de sites cliniques de créer une copie locale du modèle et de former la copie locale respective du modèle sur des données de formation au site clinique respectif ;
b) recevoir, de chacun de la pluralité de sites cliniques, i) une mise à jour locale d'un paramètre dans le modèle obtenu en formant la copie locale du modèle sur les données de formation au site clinique respectif et ii) des métadonnées fournissant une indication d'une qualité des données de formation au site clinique respectif en fournissant une indication de la manière dont les données de formation sont équilibrées au site clinique entre différentes classifications de sortie du modèle ; et
c) mettre à jour le paramètre dans le modèle global, sur la base des mises à jour locales reçues du paramètre et des métadonnées reçues, en combinant les mises à jour locales du paramètre pour déterminer une mise à jour du modèle global en pondérant chaque mise à jour locale en fonction des métadonnées respectives, de telle sorte que des mises à jour locales résultant d'ensembles de données de formation plus équilibrés reçoivent plus de poids par comparaison à des mises à jour locales résultant d'ensembles de données de formation moins équilibrés.

10. Produit de programme informatique comprenant un support lisible par ordinateur, le support lisible par ordinateur présentant un code lisible par ordinateur incorporé dans celui-ci, le code lisible par ordinateur étant configuré de sorte que, lors de son exécution par un ordinateur ou un processeur approprié, l'ordinateur ou le processeur soit amené à réaliser le procédé selon l'une quelconque des revendications 1 à 8.

Fig. 1

204 — Processor — 202

206 — Memory

Fig. 2

EP 4 179 467 B1

Send information to the plurality of clinical sites to enable each of the plurality of clinical sites to create a local copy of the model and train the respective local copy of the model on training data at the respective clinical site — 302

Receive, from each of the plurality of clinical sites, i) a local update to a parameter in the model obtained by training the local copy of the model on the training data at the respective clinical site and ii) metadata related to a quality of the training performed at the respective clinical site — 304

Update the parameter in the global copy of the model, based on the received local updates to the parameter and the received metadata — 306

Fig. 3

Fig. 4

502 — Processor

504 — Memory

500

Fig. 5

600

602
Receive information from a central server enabling a local copy of the model to be created and trained on training data at the clinical site

604
Train a local copy of the model according to the information

606
Send to the central server, i) an update to the model based on training of the local copy of the model on the training data at the clinical site and ii) metadata related to a quality of the training performed at the respective clinical site

Fig. 6

Fig. 7

Fig. 8

**EP 4 179 467 B1**

**Patent documents cited in the description**

- EP 3528179 A1

**Non-patent literature cited in the description**

- **BONAWITZ et al.** *Towards Federated Learning at Scale: System Design*, 2019